**Europäisches Patentamt**

**European Patent Office**

**Office européen des brevets**

(19)

(11) Veröffentlichungsnummer: **0 240 906 B1**

(12) # EUROPÄISCHE PATENTSCHRIFT

(45) Veröffentlichungstag der Patentschrift :
02.05.91 Patentblatt 91/18

(51) Int. Cl.⁵ : **A61J 3/10**

(21) Anmeldenummer : **87104731.2**

(22) Anmeldetag : **31.03.87**

(54) Kontinuierliches Verfahren zum Tablettieren.

(30) Priorität : **11.04.86 DE 3612211**

(43) Veröffentlichungstag der Anmeldung :
**14.10.87 Patentblatt 87/42**

(45) Bekanntmachung des Hinweises auf die
Patenterteilung :
**02.05.91 Patentblatt 91/18**

(84) Benannte Vertragsstaaten :
**AT BE CH DE ES FR GB GR IT LI NL SE**

(56) Entgegenhaltungen :
**DE-A- 1 766 546**
**FR-A- 537 444**
**US-A- 4 411 161**

(73) Patentinhaber : **BASF Aktiengesellschaft**
**Carl-Bosch-Strasse 38**
**W-6700 Ludwigshafen (DE)**

(72) Erfinder : **Klimesch, Roger Guenther, Dr.**
**Georg-Froeba-Strasse 43**
**W-6146 Alsbach/Haehnlein (DE)**
Erfinder : **Bleckmann, Gerhard**
**Giselherstrasse 9**
**W-6840 Lampertheim (DE)**
Erfinder : **Farwerck, Karl-Peter**
**Enzingerstrasse 32**
**W-6520 Worms 21 (DE)**
Erfinder : **Goertz, Hans-Helmut, Dr.**
**Am Wurmberg 11**
**W-6713 Freinsheim (DE)**
Erfinder : **Schlemmer, Lothar**
**Duisbergstrasse 1 a**
**B-6701 Maxdorf 2 (DE)**

## Beschreibung

Die Erfindung betrifft ein einfaches, kontinuierliches Verfahren zur Herstellung von Tabletten.

Die üblichen Tablettiermaschinen arbeiten im Taktverfahren mit Stempel und Matrize. Das Verfahren erfordert intensiv vorgemischte Tablettiermassen und ist daher insgesamt mehrstufig und aufwendig.

Der Erfindung lag die Aufgabe zugrunde, ein einfaches, kontinuierliches Verfahren zum Tablettieren zu entwickeln. Die Lösung dieser Aufgabe besteht in einem kontinuierlichen Verfahren zum Tablettieren von extrudierbaren pharmazeutischen Mischungen, das dadurch gekennzeichnet ist, daß man die Mischung extrudiert und den noch verformbaren Strang zwischen zwei gegenläufig angetriebenen Walzen mit einander gegenüberliegenden Vertiefungen im Walzenmantel, deren Ausführung die Tablettenform bestimmt, verpreßt. Falls halbkugelförmige oder andere Tabletten mit einer ebenen Seite hergestellt werden sollen, läßt man den einen Walzenmantel glatt.

Wenn auch Fälle denkbar sind, in denen ein Vormischen zweckmäßig ist, so daß ein einfacher Extruder genügt, so ist es doch in der Regel wesentlich vorteilhafter, wenn der Extruder in üblicher eise als ein- oder mehrwelliger Mischextruder gestaltet ist, so daß ein Vormischen nicht erforderlich ist. Der Mischextruder (1) kann mehrere Einfüllstutzen (2), gegebenenfalls für die getrennte Zugabe von festen und flüssigen Mischungsbestandteilen, sowie einen Rohrstutzen zur Inertbegasung (in der Regel Stickstoff) und/oder Entgasung besitzen. Zur Erhöhung des Durchsatzes kann der Extruder mehr als eine Düse (3) haben.

Um einen sicheren Transport des extrudierten Stranges zu gewährleisten und sein Abreißen hinter der Düse zu vermeiden, extrudiert man zweckmäßig schräg nach unten. Der jeweils zweckmäßigste Winkel hängt von den Produkteigenschaften und der Fahrweise (z.B. Extrusionstemperatur und -geschwindigkeit) ab.

Die Formgebung schließt sich unmittelbar an den Extrusionsvorgang an. Man führt den noch plastischen extrudierten Strang, gegebenenfalls mit Hilfe einer geeigneten Führungsrinne, durch ein Walzenpaar (4), wobei die Mäntel der beiden Walzen einander gegenüberliegende Vertiefungen (5) besitzen, die an der Berührungslinie paarweise die Tablettenform bilden.

In einer bevorzugten Ausführungsform, insbesondere für klebrige Massen, die mehr oder weniger stark an der Formwand (Vertiefungen 5) haften, sind die Vertiefungen durch einen offenen Kanal miteinander verbunden, so daß die geformten Tabletten (6) über einen kurzen Steg (Sollbruchstelle) (7) miteinander verbunden bleiben. Es entstehen auf diese Weise je nach Flexibilität des Materials perlenkettenartige oder starre Gebilde, die mehrere Tabletten enthalten.

Die perlenkettenartigen Gebilde können aufgewickelt und mit Hilfe relativ einfacher Maschinen rationell verpackt werden. Die starren Gebilde können auf eine gewünschte Länge oder in Einzeltabletten zerbrochen werden.

Wenn die Adhäsion der gebildeten Tabletten an den Formwänden (den Vertiefungen in den Walzenmänteln) gering genug ist, daß auch einzelne (nicht über Stege verbundene) Tabletten nicht kleben bleiben, sondern aus den Vertiefungen frei herausfallen, kann auf die erwähnten Verbindungsstege verzichtet werden. Längliche Vertiefungen (für Oblongtabletten oder Zäpfchen) werden dann vorteilhaft parallel zur Längsachse der Walze angeordnet.

Im allgemeinen genügt für die Walzen die natürliche Luftkühlung. In besonderen Fällen kann es vorteilhaft sein, sie zusätzlich zu kühlen oder auch zu heizen. Für solche Fälle sollten die Walzen zweckmäßig temperierbar sein. d.h. eine der üblichen Konstruktionen zum Durchleiten eines flüssigen Kühl- oder Heizmediums besitzen. Siehe z.B. De-A-1 766 546 : bei diesem Verfahren ist jedoch die pharmazeutische Mischung flüssig und ersfarrt nur durch die Kührung und nicht durch Druck.

Falls der Extruder mehr als eine Düse hat, gehört natürlich zu jeder Düse eine auf jedem Walzenumfang in einer Ebene senkrecht zur Achse umlaufende Reihe von formgebenden Vertiefungen (5).

Das erfindungsgemäße Verfahren ist das erste kontinuierliche Verfahren zum Tablettieren von pharmazeutischen Mischungen. Es ist nicht nur einfacher, effektiver und damit wirtschaftlicher als die übliche Herstellung über die bekannten Tablettiermaschinen, sondern hat weitere wesentliche Vorteile :

1. freiere Gestaltung der Tablettenform (z.B. sphärisch) ;

2. die Verarbeitung von klebrigem und/oder hochviskosem Material, das sich auf den üblichen Tablettiermaschinen nicht oder nur unter großen Schwierigkeiten verarbeiten läßt, ist möglich, weil das Gewicht der zusammenhängenden Tabletten die Adhäsionskraft zwischen Tablette und Formhälfte zu überwinden vermag.

Extrudierbare pharmazeutische Mischungen sind Mischungen mindestens eines pharmazeutischen Wirkstoffes mit mindestens einem für die Herstellung pharmazeutischer Tabletten üblichen Hilfsstoff, die durch Schmelzen oder Erweichen mindestens einer Komponente teigig und daher extrudierbar sind. Das sind insbesondere Mischungen, die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylacetat, Copolymerisate von Vinylacetat und Crotonsäure, teilverseiftes Polyvinylacetat, Polyvinylalkohol, Ethylen/Vinylacetat-Copolymerisate, Polyhydroxyethylmethacrylat, Copolymerisate von Methyl-methacrylat und Acrylsäure, Celluloseester, Celluloseether, Polyethylenglykol, Polyethylen. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Hasse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Bevorzugt werden NVP-Polymerisate die in Mischung mit dem Wirkstoff und ggf. üblichen galenischen Hilfsstoffen mit oder vorzugsweise ohne weichmachende Zusätze im gewünschten Temperaturbereich schmelzen oder erweichen. Das Schmelzen oder Erweichen unterhalb einer bestimmten Temperatur ist gegebenenfalls erforderlich im Hinblick auf eine mögliche thermische und/oder oxidative Schädigung nicht nur des Wirkstoffs, sondern auch des NVP-Polymerisates. Dieses könnte beim Extrudieren vergilben, weshalb die Extrusion von NPV-Polymerisaten bisher nicht üblich ist. Die Gefahr ist jedoch gering bei Extrusionstemperaturen unter 180°C, vor allem unter 130°C, wenn das Polymerisat nicht in wäßriger Lösung mit Wasserstoffperoxid als Starter hergestellt wurde, sondern in einem organischen Lösungsmittel, oder aber in Wasser mit einem organischen Peroxid als Starter, etwa nach dem Verfahren gemäß der deutschen Patentanmeldung P 36 42 633.4 oder nach dem Verfahren von US 4 520 179 und 4 520 180.

Falls der K-Wert über 17, insbesondere über 30 oder gar 40 (bis maximal 70) liegt und keine stark weichmachende Komponente zugegen ist, kommen als NVP-Polymerisate nur solche mit einer Glastemperatur Tg unter 120, vorzugsweise unter 100°C in Betracht, oder das NVP-Polymerisat (einschließlich Homopolymer) darf nicht in Wasser mit $H_2O_2$ als Starter hergestellt worden sein. Dabei würden nämlich Polymer-Endgruppen entstehen, die bei höherer Temperatur zur Vergilbung führen.

Das NVP-Polymerisat kann über die Art und Menge an Comonomeren je nach Anwendungszwerck so stark oder so schwach hydrophil eingestellt werden, daß sich die daraus hergestellten Tabletten im Mund (Buccaltablette) oder im Magen oder auch erst im Darm (rasch oder verzögert) auflösen oder so quellen, daß sie den Wirkstoff freigeben. Sie sind dann ausreichend quellbar, wenn sie bei Lagerung bei 90% relativer Luftfeuchtigkeit mehr als 10 Gew.% Wasser aufnehmen. Falls es bei carboxylgruppenhaltigen Bindemitteln erwünscht ist, daß sie erst im alkalischen Milieu des Darms den Wirkstoff freigeben, gilt die obige Angabe der Wasseraufnahme nur für die neutralisierte Form (Salzform) des Polymeren (in der die Protonen der Carboxylgruppen ganz oder teilweise durch Ammonium-, Natrium- oder Kaliumionen ersetzt sind).

Als Comonomer zum NVP kommen in Betracht : ungesättigte Carbonsäuren, z.B. Methacrylsäure, Crotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure- und Itaconsäure (wobei der Halbester vorzugsweise erst nach der Polymerisation gebildet wird), N-Vinylcaprolactam und Vinylpropionat. Bevorzugte Comonomere sind Acrylsäure und insbesondere Vinylacetat. Es werden daher NVP-Polymerisate bevorzugt, die entweder nur NVP oder Vinylacetat als einziges Comonomeres einpolymerisiert enthalten. Vinylacetat und Vinylpropionat können nach der Polymerisation ganz oder teilweise verseift sein.

Übliche galenische Hilfsstoffe, deren Gesamtmenge bis zu 100 Gew.%, bezogen auf das Polymerisat, betragen kann, sind z.B. Streckmittel wie Silicate oder Kieselerde, Stearinsäure oder deren Salze mit z.B. Magnesium oder Kalzium, Methylcellulose, Natrium-Carboxymethylcellulose, Talkum, Saccharose, Lactose, Getreide- oder Maisstärke, Kartoffelmehl, Polyvinyl-alkohol, ferner Hetz-, Konservierungs-, Spreng-, Adsorptionsmittel, Farbstoffe, Geschmacksstoffe (vgl. z.B. H. Sucker et al., Pharmazeutische Technologie, Thieme-Verlag. Stuttgart 1978).

Falls gewünscht, kann die erfindungsgemäß hergestellte Tablette auch mit einem üblichen Überzug zur Verbessserung des Ausssehens und/oder des Geschmacks (Dragee) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt. Ferner können nach dem erfindungsgemäßen Verfuhren sehr kleine Tabletten hergestellt werden, die mit Vorteil anstelle herkömmlicher Granulate in Kapseln abgefüllt werden. Der Begriff "Tablette" im Sinne der Erfindung ist weder un eine bestimmte Form noch an die perorale Anwendung gebunden. Er schließt vielmehr ruch (nicht bei Körpertemperutur schmelzende) Zäpfchen zur rektalen Anwendung ein.

Unter pharzmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.% liegen. Auch WirkstoffKombinationen können eingesetzt werden. Wirkstoffe im Sinne der Erfindung sind auch Vitamine.

Die in den Beispielen genannten Teile und Prozente beziehen sich auf das Gewicht.

Beispiel 1

45 Teile eines Copolymerisats vom K-Wert 30 aus 60 Gew.% N-Vinylpyrrolidon und 40 Gew.% Vinylacetat, 5 Teile Stearylalkohol und 50 Teile Theophyllin wurden in einem Doppelschneckenextruder vermischt und extrudiert. Die Temperatur des sechs Schüsse enthaltenden Extrudermantels betrug 30, 60, 60, 60, 60 und 60°C ; die Düse wurde auf 100°C aufgeheizt. Der hierbei erhaltene Strang wurde direkt mit dem in den Ansprüchen 2 und 3 beschriebenen Gerät zu Oblongtabletten verpreßt. Es entstanden starre Tablettenstränge, die sehr leicht in die einzelnen Tabletten zerbrachen.

Die so erhaltenen Tabletten waren gegen mechanische Einflüsse stabil und zeigten keinen Abrieb beim Transport und Verpacken.

Beispiel 2

50 Teile des Copolymerisats von Beispiel 1 und 50 Teile Theophyllin wurden in einem Doppelachneckenextruder vermischt und extrudiert. Abweichend von Beispiel 1 wurde die Temperatur der Schüsse auf 30, 60, 60, 60, 90 und 120°C eingestellt. Die Düse hatte ebenfalls eine Temperatur von 120°C. Der erhaltene Strang wurde analog zu Beispiel 1 mit dem in den Ansprüchen 2 und 3 beschriebenen Gerät zu Oblongtabletten verpreßt. Auch diese analog Beispiel 1 erhaltenen Tabletten waren gegen mechanische Einflüsse stabil.

Beispiel 3

47, 5 Teile einem Copolymerisats vom K-Wert 30 aus 60 Gew.% N-Vinylpyrrolidon und 40 Gew.% Vinylacetat, 2, 5 Teile vernetztes PVP als Tablettenprengmittel und 50 Teile Theophyllin wurden in einem Doppelschneckenextruder vermischt und extrudiert. Die Temperatur der fünf Schüsse betrug jeweils 120°C, die Düse hatte eine Temperatur von 130°C. Der noch plastische Strang wurde wie bei Beispiel 1 zu Oblongtabletten verpreßt. Die Tabletten waren stabil gegen mechanische Einflüsse.

Beispiel 4

50 Teile einem Copolymerisats vom K-Wert 52 aus 30 Gew.% N-Vinylpyrrolidon und 70 Gew.% Vinylacetat und 50 Teile Theophyllin wurden in einem Doppelschneckenextruder gemischt und extrudiert. Die Temperatur der fünf Schüsse betrug 30, 60, 100, 100 und 120°C. Die Düsse wurde ebenfalls auf 120°C aufgeheizt. Der noch plastische Strang wurde wie bei Beispiel 1 zu mechanisch stabilen Oblongtabletten verpreßt.

Beispiele 5 bis 8

Eine Mischung von 50 Gew.% eines N-Vinylpyrrolidon-Homopolymeren (PVP) von dem jeweils in der Tabelle angegebenen K-Wert und 50 Gew.% Theophyllin wurden in einem Einwellen-Extruder bei der jeweils in der Tabelle angegebenen Temperatur aufgeschmolzen, extrudiert und wie bei Beispiel 1 zu Tabletten verformt.

| Beispiel | K-Wert | T [°C] 1. | 2. | 3. | 4. | 5. | Düse |
|---|---|---|---|---|---|---|---|
| | | | | Schuß | | | |
| 5 | 12 | 115 | 125 | 135 | 135 | 135 | 145 |
| 6 | 17 | 125 | 125 | 135 | 145 | 145 | 155 |
| 7 | 25 | 145 | 155 | 165 | 175 | 175 | 175 |
| 8 | 30 | 150 | 160 | 160 | 170 | 180 | 180 |
| 8a | 60 | 150 | 160 | 160 | 170 | 180 | 180 |

Beispiel 9

40 Teile eines Copolymerisats aus 60 Gew.% N-Vinylpyrrolidon und 40 Gew.% Vinylacetat vom K-Wert 30, 10 Teile Polyhydroxyethylmethacrylat und 50 Teile Theophyllin wurden analog zu Beispiel 1 zu mechanisch stabilen Tabletten verarbeitet. Temperatur der Schüsse : 70, 80, 80, 80, 80°C. Düse : 90°C.

Beispiel 10

50 Teile eines handelsüblichen, zu 80% verseiften Polyvinylacetats und 50 Teile Theophyllin wurden analog zu Beispiel 1 verarbeitet. Die temperatur der 5 Schüsse betrug 100, 100, 110, 120, 130. Düse : 150°C.

Beispiel 11

50 Teile Polyhydroxyethylmethacrylat vom K-Wert 30 und 50 Teile theophyllin wurden analog Beispiel 1 verarbeitet. Temperatur der Schüsse 120 130, 150, 160, 160°C. Düse : 170°C.

Beispiel 12 bis 14

36 Teile eines Copolymerisates aus 60 Gew.% N-Vinylpyrrolidon und 40 Gew.% Vinylacetat vom K-Wert 30, 4 Teile Stearylalkohol, 40 Teile Theophyllin und 20 Teile
Beispiel 12) Stärke
Beispiel 13) Lactose
Beispiel 14) Saccharose wurden in einem 6-schüssigen Dopppelschneckenextruder gemischt und analog Beispiel 1 zu Tabletten verformt. Die Temperatur der Schüsse betrug 90, 100 110, 120, 120, 130°C, die der Düse 135°C.

Beispiele 15 bis 17

50 Teile des Copolymerisates der Beispiele 12 bis 14 und 50 Teile Verapamil wurden gemäß den Beispielen 12 bis 14 zu Tabletten geformt.
Analog den obigen Beispielen wurden die folgenden durchgeführt. Die Bedingungen der Verarbeitung sowie die Freisetzungsgeschwindigkeiten beim half-change-Test (vgl. R. Voigt, Lehrbuch der pharmazeutischen Technologie, 5. Aufl., Verl. Chemie, Weinheim ; Deerfield Beach, Florida ; Basel, 1984, S. 627, in Verbindung mit der Paddle-Methode nach USP 21) sind tabellarisch erfaßt.

Tabelle

| Beisp. Nr. | Wirkstoff | Polymer | Hilfs- stoff | Gew.-Verhältnis Wirkst./Polymer/ Hilfsstoff | T1 | T2 | T3 | T4 | T5 | T6 | Düse | Freisetzungs- geschwindig- keit |
|---|---|---|---|---|---|---|---|---|---|---|---|---|
| 18 | Pseudoephedrin 47,5 Diphenhydramin 2,5 | A | ./. | 50/50/0 | 60 | 80 | 100 | 120 | 120 | 120 | 120 | 100 % in 1 h |
| 19 | Propafenon | A | Stärke | 40/40/20 | 60 | 70 | 90 | 110 | 110 | 110 | 110 | 100 % in 1 h |
| 20 | Propafenon | A | StA | 60/35/5 | 80 | 90 | 100 | 120 | 140 | 140 | 140 | 100 % in 2 h |
| 21 | Propafenon | A | StA | 60/30/10 | 80 | 90 | 100 | 120 | 130 | 130 | 140 | 52 % in 6 h |
| 22 | Propafenon | A | StS | 60/35/5 | 70 | 90 | 100 | 110 | 115 | 115 | 115 | 42 % in 6 h |
| 23 | Propafenon | B | StA | 50/40/10 | 65 | 80 | 95 | 110 | 110 | 110 | 110 | 100 % in 6 h |
| 24 | Propafenon | A | MgSt | 60/35/5 | 60 | 70 | 80 | 80 | 95 | 100 | 100 | 95 % in 6 h |
| 25 | Propafenon | A | MgSt | 50/40/10 | 60 | 70 | 80 | 80 | 95 | 100 | 100 | 80 % in 6 h |
| 26 | Anipamil | A | MgSt | 50/40/10 | 30 | 30 | 40 | 40 | 60 | 60 | 60 | 100 % in 2 h |
| 27 | Vitamin B1 | B | ./. | 50/50/0 | 40 | 40 | 50 | 60 | 80 | 80 | 80 | 100 % in 1 h |
| 28 | Nicotinsäure | A | ./. | 50/50/0 | 60 | 70 | 80 | 95 | 95 | 100 | 100 | 100 % in 1 h |
| 29 | Biperiden | A | StA | 50/45/5 | 80 | 90 | 100 | 120 | 120 | 130 | 135 | 100 % in 4 h |
| 30 | Biperiden | A | ./. | 50/50/0 | 80 | 90 | 110 | 120 | 140 | 140 | 140 | 100 % in 1 h |
| 31 | Canthaxantin | B | ./. | 50/50/0 | 30 | 30 | 40 | 40 | 60 | 60 | 60 | 100 % in 1 h |
| 32 | Canthaxantin | A | ./. | 50/50/0 | 40 | 40 | 55 | 60 | 60 | 80 | 80 | 100 % in 1 h |

EP 0 240 906 B1

Tabelle - Forts.

| Beisp. Nr. | Wirkstoff | Polymer | Gew.-Verhältnis Wirkst./Polymer | T1 | T2 | T3 | T4 | T5 | T6 | Düse |
|---|---|---|---|---|---|---|---|---|---|---|
| 33 | Indomethacin | A | 25/75 | 50 | 60 | 70 | 80 | 80 | 80 | 80 |
| 34 | Indomethacin | B | 25/75 | 60 | 80 | 100 | 120 | 120 | 120 | 120 |
| 35 | Anipamil | A | 25/75 | 30 | 30 | 40 | 50 | 50 | 60 | 60 |
| 36 | Anipamil | B | 25/75 | 30 | 30 | 40 | 50 | 50 | 60 | 60 |
| 37 | Benzocain | D | 25/75 | 60 | 80 | 95 | 100 | 120 | 120 | 140 |
| 38 | Benzocain | D | 25/75 | 60 | 80 | 95 | 100 | 120 | 130 | 140 |
| 39 | Benzocain | F | 25/75 | 30 | 30 | 40 | 50 | 50 | 60 | 60 |
| 40 | Benzocain | B | 25/75 | 60 | 80 | 100 | 120 | 120 | 120 | 120 |
| 41 | 5,5-Diphenhydramin | B | 25/75 | 60 | 80 | 100 | 120 | 120 | 120 | 120 |
| 42 | Paracetamid | B | 25/75 | 60 | 80 | 100 | 120 | 120 | 120 | 120 |
| 43 | Sulfathiazol | B | 25/75 | 70 | 90 | 100 | 100 | 100 | 100 | 120 |
| 44 | Sulfathiazol | E | 25/75 | 70 | 90 | 100 | 100 | 100 | 110 | 120 |
| 45 | Benzocain | A | 25/75 | 30 | 30 | 40 | 50 | 60 | 70 | 70 |
| 46 | 5,5-Diphenhydramin | A | 25/75 | 60 | 80 | 100 | 120 | 120 | 120 | 130 |
| 47 | Paracetamol | A | 25/75 | 60 | 80 | 100 | 120 | 120 | 120 | 130 |
| 48 | Sulfathiazol | A | 50/50 | 70 | 90 | 100 | 100 | 100 | 100 | 130 |
| 49 | Vitamin C | C | 50/50 | 75 | 95 | 95 | 120 | 120 | 120 | 120 |

EP 0 240 906 B1

Tabelle - Forts.

| Beisp. Nr. | Wirkstoff | Polymer | Gew.-Verhältnis Wirkst./Polymer | T1 | T2 | T3 | T4 | T5 | T6 | Düse |
|---|---|---|---|---|---|---|---|---|---|---|
| 50 | Benzocain | E | 25/75 | 60 | 70 | 80 | 120 | 130 | 130 | 130 |
| 51 | Benzocain | G | 25/75 | 60 | 70 | 70 | 80 | 80 | 80 | 120 |
| 52 | Benzocain | H | 25/75 | 50 | 60 | 60 | 60 | 80 | 90 | 110 |
| 53 | Benzocain | I | 25/75 | 50 | 60 | 70 | 70 | 75 | 75 | 80 |

A = Copolymer aus 60 Gew.% NVP und 40 Gew.% Vinylacetat, K-Wert ca. 33

B = PVP, K-Wert 12

C = PVP, K-Wert 17

D = Mowiol® 30-92 (zu 92 % hydrolys. Polyvinylalkohol)

E = Mowiol 4-80 (zu 80 % hydrolys. Polyvinylalkohol)

F = Copolymer aus NVP, Vinylacetat und Hydroxypropylacrylat im Gewichtsverhältnis 30/40/30; K-Wert ca. 18

G = Celluloseacetat

H = Celluloseacetat-phthalat

I = Copolymer Vinylacetat/Crotonsäure; K-Wert ca. 30

StA = Stearylalkohol

StS = Stearinsäure

MgSt = Magnesiumstearat

EP 0 240 906 B1

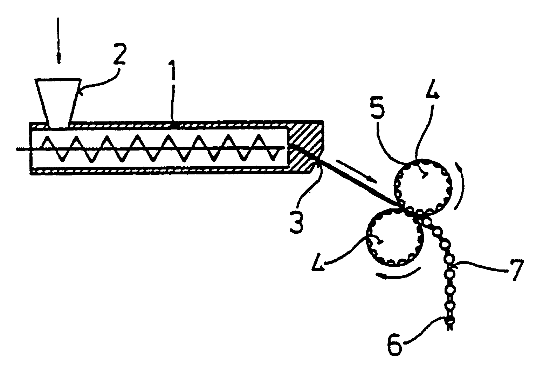

**Ansprüche**

1. Kontinuierliches Verfahren zum Tablettieren von extrudierbaren pharmazeutischen Mischungen, dadurch gekennzeichnet, daß man die Mischung extrudiert und den noch verformburen Strang zwischen zwei gegenläufig angetriebenen Walzen mit einunder gegenüberliegenden Vertiefungen im Walzenmantel, deren Ausführung die Tablettenform bestimmt, verpreßt.

2. Verfahren nach Anspruch 1, dadurch gekennzeichnet, daß man wirkstoffhaltige Polymerschmelzen extrudiert und verpreßt.

3. Verfahren nach Anspruch 1, dadurch gekennzeichnet, daß man wirkstoffhaltige Schmelzen von N-Vinylpyrrolidin-2-on-(NVP)-Polymerisaten extrudiert und verpreßt.

4. Verfahren nach Anspruch 1, dadurch gekennzeichnet, daß man wirkstoffhaltige Schmelzen von N-Vinylpyrrolidin-2-on-(NVP)-Polymerisaten extrudiert und verpreßt, wobei das NVP-Polymerisat entweder in einem organischen Lösungsmittel oder mit einem organischen Peroxid in Wasser hergestellt wurde.

5. Vorrichtung zur Durchführung des Verfahrens nach Anspruch 1, bestehend aus einem Mischextruder (1) mit einem oder mehreren Einfüllstutzen (2) und mit einer oder mehreren Strangdüsen (3) sowie zwei anschließend angeordneten gegenläufig angetriebenen Walzen (4) mit pro Düse auf jeder Walze einer in einer Ebene senkrecht zur Achse auf der Mantelfläche umlaufenden Reihe von einander gegenüberliegenden Vertiefungen (5), die an der Berührungslinie der Walzen paarweise die Tablettenform bilden.

6. Vorrichtung nach Anspruch 5, dadurch gekennzeichnet, daß die Walzen zwischen je zwei Vertiefungen einen offenen Verbindungskanal besitzen, so daß die entstehenden Tabletten (6) über Stege (7) miteinander verbunden sind.

**Claims**

1. A continuous method for tableting extrudable pharmaceutical mixtures, wherein the mixture is extruded and the still deformable extrudate is pressed between two rollers which are driven in opposite directions and possess depressions opposite one another in the roller shell, the form of these depressions determining the tablet shape.

2. A process as claimed in claim 1, wherein a polymer melt containing an active compound is extruded and pressed.

3. A process as claimed in claim 1, wherein an active compound-containing melt of an N-vinylpyrrolidin-2-one (NVP) polymer is extruded and pressed.

4. A process as claimed in claim 1, wherein an active compound-containing melt of an N-vinylpyrrolidin-2-one (NVP) polymer is extruded and pressed, the NVP polymer being prepared either in an organic solvent or using an organic peroxide in water.

5. Apparatus for carrying out the method as claimed in claim 1, consisting of a mixing extruder (1), which has one or more feed orifices (2) and one or more dies (3), and two downstream rollers (4) which are driven in opposite directions and possess, per die, a number of depressions (5) opposite one another on each roller which run around the cylinder surface in a plane at right angles to the axis and, in pairs, generate the tablet shape at the line of contact of the rollers.

6. Apparatus as claimed in claim 5, wherein the rollers possess an open connecting channel between each pair of depressions, so that the resulting tablets (6) are connected to one another via webs (7).

**Revendications**

1. Procédé continu de pastillage de mélanges pharmaceutiques extrudables, caractérisé par le fait qu'on extrude le mélange et qu'on presse le boudin encore déformable entre deux cylindres entraînés en sens inverses l'un de l'autre et dont les enveloppes sont munies de creux se faisant face et dont la forme détermine celle des pastilles ou pilules.

2. Procédé selon la revendication 1, caractérisé par le fait qu'on extrude des masses fondues de polymères à teneur en principe actif et qu'on les presse.

3. Procédé selon la revendication 1, caractérisé par le fait qu'on extrude des masses fondues, à teneur en principe actif, de polymérisats de N-vinylpyrrolidin-2-one-(NVP)- et on les presse.

4. Procédé selon la revendication 1, caractérisé par le fait qu'on extrude des masses fondues, à teneur en principe actif, de polymérisats de N-vinylpyrrolidin-2-one-(NVP)- et on les presse, le polymérisat de NVP étant préparé, soit dans un solvant organique soit avec un péroxyde organique dans l'eau.

9